# FASCICULE DE BREVET EUROPEEN

(11) **EP 0 599 687 B1**
(45) Date de publication et mention de la délivrance du brevet: **29.05.1996**
(21) Numéro de dépôt: 93402756.6
(22) Date de dépôt: 12.11.1993
(51) Int. Cl.: A61K 7/00, A61K 7/02, A61K 7/48, A61K 7/043

(54) **Composition cosmétique de maquillage contenant un fullerène ou un mélange de fullerènes**
Schminke enthaltend eine Fullerene oder eine Mischung von Fullerenen
Make up composition containing a fullerene or a mixture of fullerenes

(30) Priorité: 13.11.1992 FR 9213675
(43) Date de publication de la demande: 01.06.1994
(73) Titulaire: L'OREAL, F-75008 Paris (FR)
(72) Inventeur: Mellul, Myriam, F-94240 L'Hay les Roses (FR)
(74) Mandataire: Stalla-Bourdillon, Bernard

(56) Documents cités:
- US-A- 5 114 477
- DATABASE WPI Week 9309, Derwent Publications Ltd., London, GB; AN 93-71008 & JP-A-517 328 (KANEBO)
- DATABASE WPI Week 9309, Derwent Publications Ltd., London, GB; AN 93-71007 & JP-A-517 327 (KANEBO)

## Description

La présente invention a pour objet une composition cosmétique pour le maquillage notamment de la peau, des yeux, des ongles et des cheveux, contenant en tant que charges ou pigments un fullerène ou un mélange de fullerènes.

Les produits de maquillage du visage sont essentiellement des fards à paupières, des eye-liners, des mascaras, des poudres, des fonds de teint, des fards à joues, des "blush", des crèmes teintées, des rouges à lèvres, des sticks à lèvres ou encore des sticks anti-cernes.

Tous ces produits, ainsi d'ailleurs que les vernis à ongles, contiennent en des proportions variables des charges minérales et/ou des pigments destinés à conférer à ces produits une certaine couleur ainsi d'ailleurs que diverses propriétés cosmétiques tel qu'un bon pouvoir couvrant ainsi qu'un agrément d'application.

Parmi les charges minérales et les pigments couramment utilisés en cosmétique, on peut mentionner :
- les composés minéraux naturels broyés tels que le talc, la séricite, le mica, etc,
- les oxydes métalliques obtenus par voie synthétique ou d'origine naturelle tels que l'oxyde de fer, de titane, de zinc, de chrome, etc,
- les composés minéraux synthétiques tel que le nitrure de bore, l'oxychlorure de bismuth, le mica recouvert de dioxyde de titane, etc,
- des métaux purs tels que par exemple l'aluminium en paillettes, et
- certains composés particuliers tels que le pyrophosphate de manganèse, le bleu outremer, le bleu de Prusse, ces derniers composés étant uniquement utilisés pour leur pouvoir colorant.

Le noir de charbon, la mélanine synthétique ou naturelle sont également utilisés comme source de pigment.

Parmi l'ensemble de ces composés, les formulateurs ne disposent que de peu de pigments brun foncé à noir, ceux-ci étant essentiellement l'oxyde de fer noir, le noir de carbone et la mélanine. D'autres pigments sont parfois utilisés mais leur pouvoir pigmentaire noir est faible, par exemple le graphite.

Le noir de carbone est très utilisé dans les eye-liners mais est difficile à disperser du fait de sa faible granulométrie. Les agglomérats formés obligent à des systèmes de broyage et de dispersion efficaces ce qui se traduit souvent par des élévations de viscosité des milieux.

L'oxyde de fer noir ne présente pas de problèmes de dispersion mais son pouvoir tinctorial est moyen et il est instable à température élevée.

Il a maintenant été trouvé que les fullerènes, troisième variété organisée du carbone, les deux autres étant le diamant et le graphite, pouvaient constituer d'excellents pigments dans des compositions cosmétiques pour le maquillage.

Les fullerènes moléculaires, comme ceci est maintenant bien connu, sont constitués de sphères creuses, entièrement fermées, d'atomes de carbone contenant de 32 à 1000 ou plus atomes de carbone dans chaque sphère.

Pour une description plus détaillée, on peut se référer aux articles suivants : R.E. SMALLEY, "Supersonic Carbon Cluster Beams in Atomic and Molecular Clusters" ; E.R. BERSTEIN Ed., Physical and Theoretical Chemistry, Vol.68, Elsevier Science, New-York 1990, pp.1-68 ; R.F. CURL et al."Fullerenes", Scientific American, pp. 32-41, Octobre 1991 ; F. DIEDERICH et al., "The Higher Fullerenes : Isolation and characterization" Science, Vol. 252, pp.548-551, Avril 1991 ; et R.E. SMALLEY "Great Balls of Carbon : The story of Buckminsterfullerene", The Sciences, Vol.31, n° 2, pp. 22-28, Mars-Avril 1991.

Parmi les fullerènes, le plus typique est le C₆₀ dont la structure rappelle celle d'un ballon. D'autres fullerènes existent, en particulier le C₇₀ ainsi que le C₈₄.

Les molécules de fullerènes C₆₀ sont d'ailleurs généralement contaminées par de faibles quantités de molécules de C₇₀ et de C₈₄.

La préparation des fullerènes ainsi que leurs caractéristiques de solubilité, cristallinité et couleur ont été décrites dans de nombreuses publications et notamment par W. KRÄTSCHMER et al., Nature, Vol.347, pp.354-358, 1990 et dans Chemical and Engineering News, pp.22-25, Octobre 1990.

La présente invention a donc pour objet une composition cosmétique pour le maquillage, contenant dans un véhicule cosmétique approprié, en tant que charge et/ou pigment, un fullerène ou un mélange de fullerènes.

Les fullerènes des compositions selon l'invention sont le C₆₀, le C₇₀ et le C₈₄ et leurs mélanges.

De préférence, le fullerène est le C₆₀ contenant une faible proportion de C₇₀ ainsi qu'éventuellement de C₈₄.

La coloration des fullerènes est fonction de l'enchaînement des atomes de carbone et peut s'étendre du jaune-orangé au noir.

Indépendamment de leur utilisation en tant que pigments dans les compositions cosmétiques, les fullerènes confèrent par ailleurs de bonnes propriétés cosmétiques, notamment une bonne facilité d'étalement et une grande douceur au toucher.

Parmi les fullerènes qui peuvent être utilisés dans les compositions selon l'invention, on peut en particulier mentionner ceux fournis par la Société TEXAS FULLERENES CORPORATION, Houston, Texas, la Société MATERIAL AND ELECTROCHEMICAL RESEARCH CORPORATION, Tucson, Arizona, la Société RESEARCH MATERIALS INC., Golden, Colorado et la Société ALDRICH, cette dernière commercialisant un fullerène C₆₀/C₇₀ sous la dénomination de "Fullerite".

Les compositions cosmétiques dans lesquelles les fullerènes peuvent être employés sont essentiellement celles concernant le maquillage du visage, c'est-à-dire les fards à paupières, eye-liners, mascaras, poudres, fonds de teint, fards à joues, "blush", crèmes teintées, rouges à lèvres, sticks à lèvres ou sticks anti-cernes, mais également le maquillage des cheveux.

Les fullerènes trouvent également une application dans les vernis à ongles en milieu aqueux.

De façon préférentielle, les compositions cosmétiques selon l'invention sont plus particulièrement destinées au maquillage des yeux, c'est-à-dire sont sous la forme de fards à paupières, d'eye-liners ou de mascaras.

Dans les compositions cosmétiques selon l'invention, le fullerène ou les mélanges de fullerènes est/sont généralement présents en une teneur comprise entre 0,01 et 50 % en poids par rapport au poids total de la composition, celle-ci étant fonction de l'effet cosmétique recherché.

Les compositions cosmétiques de maquillage selon l'invention peuvent en outre contenir des pigments et/ou des charges additionnels usuels en cosmétique.

Parmi les pigments minéraux, on peut citer, à titre d'exemples :
- le dioxyde de titane (rutile ou anatase) éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome hydraté (CI 77289) ;
- le bleu ferrique (CI 77510) ;
et tous les pigments minéraux ayant subi un traitement de surface minéral ou organique.

Parmi les pigments organiques, on peut citer, en particulier, les suivants :
- D & C red n° 19 (CI 45170) ;
- D & C red n° 9 (CI 15585) ;
- D & C red n° 21 (CI 45380) ;
- D & C orange n° 4 (CI 15510) ;
- D & C orange n° 5 (CI 45370) ;
- D & C red n° 27 (CI 45410) ;
- D & C red n° 13 (CI 15630) ;
- D & C red n° 7 (CI 15850-1) ;
- D & C red n° 6 (CI 15850-2) ;
- D & C yellow n° 5 (CI 19140) ;
- D & C red n° 36 (CI 12085) ;
- D & C orange n° 10 (CI 45425) ;
- D & C yellow n° 6 (CI 15985) ;
- D & C red n° 30 (CI 73360) ;
- D & C red n° 3 (CI 45430) ;
- le noir de carbone (CI 77266) ; et
- les laques à base de carmin de cochenille (CI 75470).

On peut utiliser également des pigments nacrés qui peuvent être choisis notamment parmi :
- les pigments nacrés blancs, tels que le mica recouvert d'oxyde de titane, l'oxychlorure de bismuth ; et
- les pigments nacrés colorés, tels que le mica titane avec des oxydes de fer, le mica titane avec du bleu ferrique, ou de l'oxyde de chrome, le mica titane avec un pigment organique de type précité, ainsi que ceux à base d'oxychlorure de bismuth,
- et tous les pigments organiques ayant subi un traitement de surface minéral ou organique.

Selon l'invention les pigments additionnels peuvent représenter de 0,01 à 90 % en poids de la composition, de préférence de 0,5 à 30 %.

Les charges sont choisies notamment parmi :
- la silice,
- le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoelithe, lépidolithe, biotite) ou d'origine synthétique. Les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de forme isotrope ayant des dimensions généralement inférieures à 30 µm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 µm dans le cas de l'oxyde de titane) : ces oxydes ont un toucher onctueux, un bon pouvoir couvrant et une opacité importante ;
- le nitrure de bore ;
- le carbonate de calcium précipité qui, sous forme de particules de dimensions inférieures à 10 µm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, etc. Ces savons, présents généralement sous la forme de particules ayant des dimensions inférieures à 10 µm ont un toucher onctueux et facilitent l'adhérence de la poudre sur la peau ;
- les poudres de polymères synthétiques, tels que le polyéthylène, les polyesters (par exemple isophtalate ou téréphtalate de polyéthylène), les polyamides, sous la forme de particules ayant des dimensions inférieures à 50 µm, qui possèdent des propriétés absorbantes, et permettent de conférer à la peau un aspect velouté, les poudres de méthacrylate ;
- des microsphères creuses, telles que par exemple les microsphères creuses d'Expancel vendues par la Société KEMANORD PLAST AB et décrites dans le brevet français 86 09289 (2.600.532).

Selon l'invention les charges additionnelles peuvent représenter de 0,01 à 90 % en poids de la composition.

Les compositions selon la présente invention peuvent notamment se présenter sous forme d'émulsion huile-dans-eau ou eau-dans-huile, ou sous forme d'une suspension en milieu solvant, ou encore sous forme de poudre libre, de poudre compactée ou de pâte anhydre. Les modes opératoires pour la préparation de ces différents types de compositions sont bien connus de l'homme de l'art.

Lorsqu'elles sont utilisées sous forme d'émulsion, les compositions peuvent contenir des agents tensio-actifs bien connus dans l'état de la technique. Ces tensio-actifs peuvent constituer de 0,01 à 30 % en poids de la composition.

Une réalisation particulièrement préférée consiste à préparer des émulsions anioniques ou non-ioniques en utilisant des agents tensio-actifs anioniques ou non-ioniques dans des proportions de préférence comprises entre 2 et 30 % en poids par rapport au poids total de la composition.

Parmi les agents tensio-actifs anioniques qui peuvent être utilisés seuls ou en mélange, on peut citer en particulier les sels alcalins, les sels d'ammonium, les sels d'amines ou les sels d'aminoalcools des composés suivants :
- les alcoylsulfates, alcoyléther sulfates, alcoylamides sulfates et éthers sulfates, alcoylarylpolyéthersulfates, monoglycérides sulfates,
- les alcoylsulfonates, alcoylamides sulfonates, alcoylarylsulfonates,α-oléfines sulfonates, paraffines sulfonates,
- les alcoylsulfosuccinates, alcoyléthersulfosuccinates, alcoylamides sulfosuccinates,
- les alcoylsulfosuccinamates,
- les alcoylsulfoacétates, les alcoylpolyglycérol carboxylates,
- les alcoylphosphates/alcoylétherphosphates,
- les acylsarcosinates, alcoylpolypeptidates, alcoylamidopolypeptidates, acyliséthionates, alcoyltaurates.

Le radical alcoyle ou acyle dans tous ces composés désigne généralement une chaîne de 12 à 18 atomes de carbone.

D'autres agents tensio-actifs anioniques sont constitués par les sels d'acides gras tels que les acides oléique, ricinoléique, palmitique, stéarique, les acides d'huile de coprah ou d'huile de coprah hydrogénée et notamment les sels d'amines tels que les stéarates d'amines.

On peut également citer :
- les acyl lactylates dont le radical acyle comprend de 8 à 20 atomes de carbone,
- les acides carboxyliques d'éthers polyglycoliques répondant à la formule :

   Alk - (OCH₂-CH₂)ₙ- OCH₂ - COOH

   sous forme acide ou salifiée où le substituant Alk correspond à une chaîne linéaire ayant de 12 à 18 atomes de carbone et où n est un nombre entier compris entre 5 et 15.

Parmi les tensio-actifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone. On peut également citer des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose.

D'autres composés entrant dans cette classe sont les produits de condensation d'un α-diol, d'un monoalcool, d'un alcoylphénol, d'un amide ou d'un diglycolamide avec le glycidol ou un précurseur de glycidol.

Les tensio-actifs non-ioniques principalement utilisés sont des alcools polyéthoxylés ou polyglycérolés tels que les alcools stéarylique, cétylstéarylique ou oléique polyéthoxylés.

Les tensio-actifs anioniques préférentiellement utilisés sont des stéarates d'amines.

Les compositions selon l'invention peuvent également se présenter sous forme d'un gel, d'une solution aqueuse ou hydroalcoolique d'un ou plusieurs polymères hydrosolubles tels que les dérivés de l'acide polyacrylique ou sous forme de gels émulsionnés obtenus par dispersion d'huiles dans des gels à l'aide d'émulsionnants tels que les "Pemulens" de la Société GOODRICH.

Les compositions conformes à la présente invention peuvent contenir, en plus des composants mentionnés ci-dessus, des ingrédients classiquement utilisés, dans les compositions cosmétiques, et choisis parmi les adoucissants, les conservateurs, les séquestrants, les parfums, les épaississants, les agents de cohésion, les polymères ainsi que les agents alcalinisants ou acidifiants habituellement utilisés dans le domaine cosmétique, les hydratants et les actifs hydrosolubles.

Les épaississants utilisables peuvent être naturels ou synthétiques. Parmi les épaississants naturels, on peut citer les gommes de diverses sortes telles que les gommes arabique, de guar ou de caroube. Parmi les épaississants de synthèse, on peut citer les dérivés cellulosiques comme l'hydroxyéthylcellulose, la carboxyméthylcellulose, les dérivés de l'amidon, les dérivés d'éthers de cellulose possédant des groupes ammonium quaternaires, les polysaccharides cationiques, les sels de polymères acryliques ou méthacryliques, les polyènes ou les polysiloxanes.

On peut également obtenir un épaississement des compositions par mélange de polyéthylèneglycol et de stéarate et/ou de distéarate de polyéthylèneglycol ou par un mélange d'esters phophoriques et d'amides gras.

Selon l'invention la phase huileuse peut représenter de 0,1 à 50 % en poids de l'émulsion.

Elle peut être constituée d'huiles, et/ou de cires.

Les cires et les huiles peuvent être d'origine végétale, animale, minérale ou synthétique.

Parmi les huiles végétales, on peut citer l'huile de jojoba, l'huile d'olive, l'huile d'amande douce,l'huile d'avocat, l'huile de coco, l'huile de germe de blé, l'huile de maïs, l'huile de palme, l'huile de sésame, l'huile de soja, l'huile d'argan, l'huile d'onagre, l'huile de bourrache et les huiles essentielles.

Parmi les huiles animales on peut notamment citer l'huile de poisson.

Parmi les huiles minérales on peut citer l'huile de vaseline et d'isohexadécane.

Parmi les huiles synthétiques on peut mentionner les palmitates d'éthyle et d'isopropyle, le palmitate d'éthyl-2 hexyle, les myristates d'alkyle tels que le myristate d'isopropyle, de butyle, de cétyle, le stéarate d'hexyle, les triglycérides des acides octanoïque et décanoïque, le ricinoléate de cétyle et l'octanoate de stéaryle, les huiles de silicone, les huiles perfluorées, les huiles de silicone fluorées.

La phase huileuse peut par ailleurs contenir des colorants, des filtres solaires, des antioxydants, des conservateurs et des principes actifs lipophiles.

Selon l'invention les compositions anhydres qui peuvent se présenter sous forme de poudre libre ou compactée, de fard solide, pâteux ou liquide peuvent contenir un liant qui peut représenter de préférence de 0,01 à 95 % en poids.

Parmi les agents liants, on peut citer notamment les huiles animales, végétales ou synthétiques, les mélanges d'huile(s) et de cire(s) et en particulier l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, etc... ; des huiles d'hydrocarbures, telles que des huiles de paraffine, le squalane, la vaseline, etc... ; des esters tels que le myristate d'isopropyle, le palmitate d'isopropyle, le stéarate de butyle, le laurate d'hexyle, l'isononanate d'isononyle, le palmitate de 2-éthyl hexyle, le laurate de 2-hexyl-décyle, le palmitate de 2-octyl-décyle, le myristate de 2-octyl-dodécyle, le succinate de di(éthyl-2-hexyle), le malate de diisostéaryle, le lactate de 2-octyl-dodécyle, le triisostéarate de glycérine, le triisostéarate de diglycérine, etc... ; des huiles de silicone comme les poly-méthylsiloxanes, les poly-méthylphénylsiloxanes, des polysiloxanes modifiés par des acides gras, des polysiloxanes modifiés par des alcools gras, des polysiloxanes modifiés par des polyoxyalkylènes, des silicones fluorées, etc... ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, etc... ; des alcools gras supérieurs tels que le cétanol, l'alcool stéarylique, l'alcool oléique, etc... ; les cires peuvent être choisies notamment parmi la cire de carnauba, la cire de candelilla, la cire d'abeille, la cire de baleine, des lanolines, des cires microcristallines, etc...

Le liant peut contenir en outre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 0,5.102Pa).

Parmi les huiles volatiles pouvant être présentes comme agents d'étalement dans la composition de l'invention, on citera par exemple les huiles de silicone telles que l'hexaméthyldisiloxane, le cyclopentadiméthyl- siloxane, le cyclotétraméthylsiloxane, des huiles fluorées comme celle commercialisée sous la dénomination GALDEN (MONTEFLUOS) ou des huiles isoparaffiniques telles que celles commercialisées sous la dénomination ISOPAR (E, G, L ou H).

Comme mentionné ci-dessus, les compositions selon l'invention peuvent également se présenter sous forme d'un vernis à ongles en milieu aqueux contenant de 0,01 à 10 % en poids de fullerène ou d'un mélange de fullerènes.

Les vernis à ongles comprennent une dispersion aqueuse filmogène synthétique contenant une substance miscible ou dispersible dans l'eau à laquelle peuvent s'ajouter une matière filmogène et les additifs usuels tels qu'un épaississant, un régulateur de pH, un réticulant, un anti-mousse, etc...

Comme dispersion aqueuse synthétique, on peut, entre autre, utiliser des dispersions d'acétate de polyvinyle, de polyuréthane, de polymères ou copolymères acryliques et de copolymères d'acétate de polyvinyle.

Selon l'invention, la dispersion aqueuse synthétique représente environ de 10 à 80 % en poids du vernis.

La matière filmogène est généralement présente à une concentration comprise entre 5 et 20 % en poids par rapport au poids total du vernis.

Parmi les matières filmogènes on peut notamment citer les dérivés de cellulose solubles dans l'eau.

Les vernis selon l'invention peuvent également contenir une résine généralement présente à une concentration comprise entre 0,5 et 15 % en poids par rapport au poids total du vernis.

Parmi les résines utilisables on peut notamment citer les résines du type acrylique, styrénique, acrylate-styrénique et vinylique.

Les vernis à ongles selon l'invention peuvent également contenir des adjuvants couramment utilisés dans les vernis à ongles tels que par exemple des filtres U.V.

On va maintenant donner à titre d'illustration plusieurs exemples de compositions cosmétiques de maquillage selon l'invention.

### EXEMPLE 1 : Mascara sous forme d'émulsion

| | |
|---|---|
| Stéarate de triéthanolamine | 15,0 g |
| Cire d'abeilles | 8,0 g |
| Paraffine | 3,0 g |
| Colophane | 2,0 g |
| Ozokérite | 10,0 g |
| Parahydroxybenzoate de propyle | 2,0 g |
| Parahydroxybenzoate de méthyle | 2,0 g |
| Gomme arabique | 0,5 g |
| Hydroxyéthylcellulose | 1,0 g |
| Fullerène C₆₀/C₇₀ (Fullerite vendue par la Société ALDRICH) | 5,0 g |
| Eau qsp | 100,0 g |

Ce mascara appliqué sur les cils donne un maquillage brun foncé.

### EXEMPLE 2 : Eye-liner sous forme de suspension

| | |
|---|---|
| Copolymère vinylpyrrolidone/Acétate de vinyle | 12,5 g |
| Alcool polyvinylique | 0,5 g |
| Alcool oléïque condensé avec 15 moles d'oxyde d'éthylène | 2,5 g |
| Propylène glycol | 7,5 g |
| Alcool éthylique | 3,0 g |
| Polyacrylate - sel de sodium | 0,5 g |
| Parahydroxybenzoate de méthyle | 0,3 g |
| Fullerène C₆₀/C₇₀/C₈₄ (vendu par TEXAS FULLERENE CORP.) | 6,0 g |
| Eau qsp | 100,0 g |

Le maquillage conféré par l'eye-liner est brun foncé.

### EXEMPLE 3 : Rouge à lèvres

| | |
|---|---|
| Butylhydroxytoluène | 0,15 g |
| Lanoline | 17,50 g |
| Cire microcristalline | 15,00 g |
| Triglycérides capryliques/capriques | 11,00 g |
| Béhénate d'octyl glycéryle | 11,00 g |
| Micatitane | 6,00 g |
| Fullerène C₆₀/C₇₀ (Fullerite vendu par la Société ALDRICH) | 0,30 g |
| Oxydes de fer | 2,70 g |
| Huile de ricin qsp | 100,0 g |

La couleur du rouge à lèvres est brun-rouge.

### EXEMPLE 4 : Poudre visage compacte

| | |
|---|---|
| Poudre de polyéthylène | 5,00 g |
| Mica | 12,00 g |
| Myristate d'isopropyle | 1,50 g |
| Huile de vaseline | 1,50 g |
| Oxyde de fer jaune | 0,80 g |
| Oxyde de fer orange | 0,60 g |
| Fullerène C₆₀/C₇₀/C₈₄ (vendu par TEXAS FULLERENES CORP.) | 0,15 g |
| Talc qsp | 100,0 g |

La poudre compacte a une teinte marron.

### EXEMPLE 5 : Gel-crème teinté

| | |
|---|---|
| Copolymère acide acrylique/alkylacrylate C₁₀-C₃₀ (vendu sous la dénomination de "PERMULEN-TR-1" par la Société GOODRICH) | 0,10 g |
| CARBOPOL 940 de la Société GOODRICH | 0,60 g |
| Triéthanolamine | 0,80 g |
| Glycérine | 3,00 g |
| Conservateur | 0,20 g |
| Cyclométhicone | 25,00 g |
| Nylon 12 | 5,00 g |
| Oxydes de fer | 0,75 g |
| Fullerene C₆₀/C₇₀/C₈₄ (vendu par TEXAS FULLERENES CORP.) | 0,10 g |
| Oxyde de titane | 4,15 g |
| Eau qsp | 100,0 g |

Le gel obtenu est de couleur marron.

### EXEMPLE 6 : Fond de teint

| | |
|---|---|
| Stéarate de glycéryle | 2,20 g |
| Triglycérides des acides caprylique/caprique | 15,00 g |
| Parahydroxybenzoate de méthyle | 0,10 g |
| Parahydroxybenzoate de propyle | 0,10 g |
| Imidazolidinyl urée | 0,30 g |
| 2-hydroxy 4-méthoxy benzophénone | 0,50 g |
| Diméthyl p-aminobenzoate d'éthyl-2 hexyle (vendu sous la dénomination de "ESCALOL 507" par la Société VAN DYK) | 0,50 g |
| Silicate de magnésium et d'aluminium | 1,00 g |
| Triéthanolamine | 1,00 g |
| Carboxyméthylcellulose | 0,16 g |
| Aluminum starch octénylsuccinate (vendu par la Société NATIONAL STARCH sous la dénomination de "DRY FLO" | 5,00 g |
| Cyclométhicone | 10,00 g |
| Propylène glycol | 2,00 g |
| Glycérine | 3,00 g |
| Lauroyl sarcosinate de sodium | 0,60 g |
| Acide stéarique | 2,20 g |
| Oxyde de fer jaune | 2,00 g |
| Oxyde de fer rouge | 1,40 g |
| Fullerène C₆₀/C₇₀ (Fullerite vendue par la Société ALDRICH) | 0,60 g |
| Oxyde de titane | 8,00 g |
| Eau qsp | 100 g |

Le fond de teint est de couleur marron.

## Revendications

1. Composition cosmétique pour le maquillage, caractérisée par le fait qu'elle contient, dans un véhicule approprié, en tant que charge et/ou pigment, un fullerène ou un mélange de fullerènes.

2. Composition cosmétique selon la revendication 1, caractérisée par le fait que le fullerène est choisi parmi le C₆₀, C₇₀ et C₈₄ atomes de carbone et leurs mélanges.

3. Composition cosmétique selon la revendication 1, caractérisée par le fait que le fullerène est le C₆₀ atomes de carbone.

4. Composition cosmétique selon l'une quelconque des revendications précédentes, caractérisée par le fait que le mélange de fullerènes est le C₆₀/C₇₀ atomes de carbone.

5. Composition cosmétique selon l'une quelconque des revendications précédentes, caractérisée par le fait qu'elle contient le fullerène ou le mélange de fullerènes en une proportion comprise entre 0,01 et 50 % en poids par rapport au poids total de la composition.

6. Composition cosmétique selon l'une quelconque des revendications précédentes, caractérisée par le fait qu'elle contient en outre un pigment additionnel à une concentration comprise entre 0,01 et 90 % en poids par rapport au poids total de la composition.

7. Composition cosmétique selon l'une quelconque des revendications précédentes, caractérisée par le fait qu'elle contient en outre au moins une charge additionnelle présente à une concentration comprise entre 0,01 et 90 % en poids par rapport au poids total de la composition.

8. Composition cosmétique selon l'une quelconque des revendications précédentes, caractérisée par le fait qu'elle se présente sous forme d'une émulsion huile-dans-eau ou eau-dans-huile, d'une suspension, ou sous forme de poudre libre, de poudre compactée, de solide ou de pâte anhydre ou encore sous forme d'un vernis à ongles aqueux.

9. Composition cosmétique selon la revendication 8, caractérisée par le fait que l'émulsion huile-dans-eau ou eau-dans-huile contient un agent tensio-actif en une proportion comprise entre 0,01 et 30 % en poids, la phase huileuse de l'émulsion étant comprise entre 0,1 et 50 % en poids par rapport au poids total de la composition.

10. Composition cosmétique selon la revendication 8, caractérisée par le fait qu'elle se présente sous forme solide ou pâteuse anhydre et contient au moins un agent liant présent en une proportion comprise entre 0,01 et 95 % en poids par rapport au poids total de la composition.

11. Composition cosmétique selon l'une quelconque des revendications 1 à 8, caractérisée par le fait qu'elle se présente sous forme d'un vernis à ongles aqueux contenant :
- de 10 à 80 % en poids d'une dispersion synthétique,
- de 5 à 20 % en poids d'une matière filmogène, et
- de 0,5 à 15 % en poids d'une résine.

12. Composition selon la revendication 11, caractérisée par le fait qu'elle contient de 0,01 à 10 % en poids de fullerène ou d'un mélange de fullerènes.

13. Utilisation d'un fullerène ou d'un mélange de fullerènes en tant que charge et/ou pigment dans un produit de maquillage.

## Claims

1. Cosmetic make-up composition, characterized in that it contains, in a suitable vehicle, as a filler and/or pigment, a fullerene or mixture of fullerenes.

2. Cosmetic composition according to Claim 1, characterized in that the fullerene is chosen from C₆₀, C₇₀ and C₈₄ carbon atoms and mixtures thereof.

3. Cosmetic composition according to Claim 1, characterized in that the fullerene is C₆₀ carbon atoms.

4. Cosmetic composition according to any one of the preceding claims, characterized in that the mixture of fullerenes is C₆₀/C₇₀ carbon atoms.

5. Cosmetic composition according to any one of the preceding claims, characterized in that it contains the fullerene or mixture of fullerenes in a proportion of between 0.01 and 50 % by weight relative to the total weight of the composition.

6. Cosmetic composition according to any one of the preceding claims, characterized in that it contains, furthermore, an additional pigment at a concentration of between 0.01 and 90 % by weight relative to the total weight of the composition.

7. Cosmetic composition according to any one of the preceding claims, characterized in that it contains, furthermore, at least one additional filler present at a concentration of between 0.01 and 90 % by weight relative to the total weight of the composition.

8. Cosmetic composition according to any one of the preceding claims, characterized in that it takes the form of an oil-in-water or water-in-oil emulsion or a suspension, or the form of a free powder or compacted powder or solid or anhydrous paste, or alternatively the form of an aqueous nail varnish.

9. Cosmetic composition according to Claim 8, characterized in that the oil-in-water or water-in-oil emulsion contains a surfactant in a proportion of between 0.01 and 30 % by weight, the oily phase of the emulsion being between 0.1 and 50 % by weight relative to the total weight of the composition.

10. Cosmetic composition according to Claim 8, characterized in that it takes the form of a solid or an anhydrous paste, and contains at least one binding agent present in a proportion of between 0.01 and 95 % by weight relative to the total weight of the composition.

11. Cosmetic composition according to any one of Claims 1 to 8, characterized in that it takes the form of an aqueous nail varnish containing:
- from 10 to 80 % by weight of a synthetic dispersion,
- from 5 to 20 % by weight of a film-forming material, and
- from 0.5 to 15 % by weight of a resin.

12. Composition according to Claim 11, characterized in that it contains from 0.01 to 10 % by weight of fullerene or of a mixture of fullerenes.

13. Use of a fullerene or mixture of fullerenes as a filler and/or pigment in a make-up product.

## Patentansprüche

1. Kosmetische Zubereitung zum Schminken, dadurch gekennzeichnet, daß sie in einem geeigneten Träger als Hilfsstoff und/oder Pigment ein Fulleren oder eine Mischung von Fullerenen enthält.

2. Kosmetische Zubereitung gemäß Anspruch 1, dadurch gekennzeichnet, daß das Fulleren aus den C₆₀, C₇₀ und C₈₄-Fullerenen und deren Gemischen ausgewählt ist.

3. Kosmetische Zubereitung gemäß Anspruch 1, dadurch gekennzeichnet, daß das Fulleren ein C₆₀-Fulleren ist.

4. Kosmetische Zubereitung gemäß einem der vorstehenden Ansprüche, dadurch gekennzeichnet, daß das Fullerengemisch aus C₆₀-/C₇₀-Fullerenen besteht.

5. Kosmetische Zubereitung gemäß einem der vorstehenden Ansprüche, dadurch gekennzeichnet, daß sie das Fulleren oder die Mischung von Fullerenen in einer Menge von 0,01 bis 50 Gew.-%, bezogen auf das Gesamtgewicht der Zubereitung, enthält.

6. Kosmetische Zubereitung gemäß einem der vorstehenden Ansprüche, dadurch gekennzeichnet, daß sie zusätzlich ein weiteres Pigment in einer Konzentration von 0,01 bis 90 Gew.-%, bezogen auf das Gesamtgewicht der Zubereitung, enthält.

7. Kosmetische Zubereitung gemäß einem der vorstehenden Ansprüche, dadurch gekennzeichnet, daß sie zusätzlich mindestens einen weiteren Hilfsstoff in einer Konzentration von 0,01 bis 90 Gew.-%, bezogen auf das Gesamtgewicht der Zubereitung, enthält.

8. Kosmetische Zubereitung gemäß einem der vorstehenden Ansprüche, dadurch gekennzeichnet, daß sie in Form einer Öl-in-Wasser- oder Wasser-in-Öl-Emulsion, einer Suspension oder in Form eines losen Puders, eines Kompaktpuders, eines/einer wasserfreien Feststoffs oder Paste oder auch in Form eines wäßrigen Nagellackes vorliegt.

9. Kosmetische Zubereitung gemäß Anspruch 8, dadurch gekennzeichnet, daß die Öl-in-Wasser- oder die Wasser-in-Öl-Emulsion ein Tensid in einer Menge von 0,01 bis 30 Gew.-% enthält und die Ölphase der Emulsion zwischen 0,1 und 50 Gew.-%, bezogen auf das Gesamtgewicht der Zubereitung, ausmacht.

10. Zubereitung gemäß Anspruch 8, dadurch gekennzeichnet, daß sie in wasserfreier fester oder pastöser Form vorliegt und mindestens ein Bindemittel in einer Menge von 0,01 bis 95 Gew.-%, bezogen auf das Gesamtgewicht der Zubereitung, enthält.

11. Kosmetische Zubereitung gemäß einem der Ansprüche 1 bis 8, dadurch gekennzeichnet, daß in Form eines wäßrigen Nagellackes vorliegt, der
- 10 bis 80 Gew.-% einer synthetischen Dispersion,
- 5 bis 20 Gew.-% eines filmbildenden Materials und
- 0,5 bis 15 Gew.-% eines Harzes
enthält.

12. Zubereitung gemäß Anspruch 11, dadurch gekennzeichnet, daß sie 0,01 bis 10 Gew.-% an Fulleren oder einer Mischung aus Fullerenen enthält.

13. Verwendung eines Fullerens oder einer Mischung aus Fullerenen als Hilfsstoff und/oder Pigment in einem Produkt zum Schminken.
